(19) Europäisches Patentamt
European Patent Office
Office européen des brevets

(11) Publication number: **0 391 319 A1**

(12) **EUROPEAN PATENT APPLICATION**

(21) Application number: **90106308.1**

(51) Int. Cl.5: **C12N 15/30, A61K 39/002**

(22) Date of filing: **02.04.90**

(30) Priority: **03.04.89 JP 84275/89**

(43) Date of publication of application: **10.10.90 Bulletin 90/41**

(84) Designated Contracting States: **AT BE CH DE DK ES FR GB GR IT LI LU NL SE**

(71) Applicant: **MOCHIDA PHARMACEUTICAL CO., LTD.**
**7, Yotsuya 1-chome**
**Shinjuku-ku Tokyo 160(JP)**

(72) Inventor: **Matsuura, Tadashi**
**3-7-4 Arigasaki**
**Matsumoto-shi, Nagano(JP)**
Inventor: **Sugane, Kazuo**
**3-4-14, Kiri**
**Matsumoto-si, Nagano(JP)**

(74) Representative: **Henkel, Feiler, Hänzel & Partner**
**Möhlstrasse 37**
**D-8000 München 80(DE)**

# (54) DNA sequence originated from toxoplasma gondii and protein encoded by said DNA sequence.

(57) A DNA sequence coding for an antigenic protein originated from and specific to *Toxoplasma. gondii* and an antigenic protein and its amino acid sequence encoded by the DNA sequence.

The antigenic protein is capable of undergoing a specific immune reaction with an anti-*Toxoplasma gondii* antiserum.

The antigenic protein is produced in a recombinant host cell by means of recombinant DNA technology, and the antigenic protein thus prepared contains substantially no other proteins originated from *Toxoplasma gondii.*

EP 0 391 319 A1

# DNA Sequence Originated from *Toxoplasma gondii* and Protein Encoded by said DNA Sequence

## BACKGROUND OF THE INVENTION

This invention relates to a gene coded for an antigenic protein which is specific for an antiserum prepared against a protozoan parasite, *Toxoplasma gondii* (to be referred to as *T. gondii* hereinafter), and a *T. gondii*-specific antigenic protein encoded by said gene.

Toxoplasmosis is an infectious disease caused by a protozoan parasite, *T. gondii*. This parasite is distributed widely in many animals, including mammals and birds, and possessed of a nature of invading various tissue cells and propagating inside the cells. This organism has especially high affinities to central nervous system cells and reticuloendothelial system cells and has been long recognized to cause various clinical symptoms, such as inflammations, including exudative retinochoroiditis, lymphadenitis and encephalitis, fever and other clinical symptoms.

In the case of human beings, it has been considered that the frequency of infection by this parasite is generally high, but the case of onset of illness is relatively rare. In spite of such a general consideration, there are many cases of the onset of illness caused by this protozoan parasite in patients who are suffering from immunodeficiency syndrome or have been treated with a drug which has an immunosuppressive reaction, such as a case of cancer and a patient who underwent a transplantation operation of an organ or bone marrow. In addition, onset of a transplacental infection has been confirmed by animal experiments. Even in human beings, pregnancy abnormalities (stillbirth and miscarriage) and the birth of congenital abnormal child caused by a fetal infection have been reported, which occurred when immunity in the mother's body has decreased, inside the womb or the placenta has been parasitized by the protozoan parasite or a pregnant woman has been infected for the first time by this parasite.

Under these circumstances, inspections for the presence of *T. gondii* infection in, especially, pregnant women are being performed at many medical organizations. A dye test, which is one of the inspection methods, is the most reliable method because of its high specificity and sensitivity. The dye test, however, is quite difficult to be performed at medical facilities, except for a special research organization, because it requires many complex manual handlings. In consequence, search for a new method which can be used in place of the dye test has been made and, as the results, diagnostic agents have been developed making use of an indirect hemagglutination (IHA), an indirect latex agglutination (ILA), or the like and are now being on the market and used commonly.

For the use of the current IHA- or ILA-aided diagnostic agent, however, *T. gondii* tachyzoites themselves or crude extracts obtained from the tachyzoites are required as the antigen in the reaction. As the results, it has been indicated that these tests have relatively high frequencies of false positive and/or false negative reactions due to non-specific reactions and are low in the accuracy of diagnosis. Since, as described above, *T. gondii* infection has a danger of increasing possibility of the birth of congenital abnormal child, a result of the infection inspection is an important indicator for deciding whether the pregnancy should be continued or not. Therefore, a low accuracy of the diagnosis becomes very serious problem for medical treatments. In addition, a large scale culturing of *T. gondii* tachyzoites is inevitably required in the production process of the diagnostic agents, which has a possible danger of causing infection of the parasite upon process workers. Because of the above reasons, there are great demands for the development of a diagnostic agent having more higher specificity to *T. gondii* and more reliable accuracy of diagnosis from the medical sites and a more safe process for producing the antigen from the production sites.

The present inventors have already searched for an antigen specific to *T. gondii* which is capable of undergoing a specific immune reaction with an anti-*T. gondii* antiserum obtained from a patient of *T. gondii* infection and found an antigenic protein which had a molecular weight of about 27,000 daltons and was capable of undergoing specific immune reaction with the anti-*T. gondii* antiserum in *in vitro* translation products of total RNA extracted from tachyzoites of *T. gondii* RH strain (The 57th Annual Meeting of The Japanese Society of Parasitology, 1988).

## SUMMARY OF THE INVENTION

On the basis of the just described discovery, the present inventors have continued the study in earnest and, as the results, have isolated a gene encoding an antigenic determinant group of a *T. gondii* tachyzoites-originated protein having a high antigenicity specific to *T. gondii,* determined base sequence of the gene and further determined amino acid sequence of the protein based on the thus determined base sequence. The present invention has been accomplished as a result of these efforts.

As have been described in the foregoing, according to the present invention, there is provided a DNA sequence coding for an antigenic protein originated from and specific to *T. gondii* tachyzoites. Also provided is a *T. gondii*-specific antigenic protein encoded by said DNA sequence.

Particularly, in accordance with the present invention, there is provided a DNA sequence represented by the following sequence [I] which encodes an antigenic protein originated from and specific to *Toxoplasma gondii* tachyzoites:

GCT TAC GCT GCC GAA GGC GGC GAC AAC CAG
TCG AGC GCC GTC TCA GAT CGG GCG TCT CTC
TTT GGT TTG CTG AGT GGA GGG ACA GGG CAG
GGA TTA GGA ATC GGA GAA TCT GTA GAT TTG
GAG ATG ATG GGG AAC ACG TAT CGT GTG GAG
AGA CCC ACA GGC AAC CCG GAC TTG CTC AAG
ATC GCC ATT AAA GCT TCA GAT GGA TCG TAC
AGC GAA GTC GGC AAT GTT AAC GTG GAG GAG
GTG ATT GAT ACT ATG AAA AGC ATG CAG AGG
GAC GAG GAC ATT TTC CTT CGT GCG TTG AAC
AAA GGC GAA ACA GTC GAG GAA GCG ATC GAA
GAC GTG GCT CAA GCA GAA GGG CTT AAT TCG
GAG CAA ACC CTG CAA CTG GAA GAT GCA GTG
AGC GCG GTG GCG TCT GTT GTT CAA GAC GAG
ATG AAG GTG ATC GAC GAT GTG CAG CAG CTT
GAA AAG GAC AAA CAA CAG CTT AAG GAT GAC
ATT GGG TTC CTA ACA GGA GAG AGA GAG TAA
TTGGAGGTAGCTTTGTTGTGATCGTACCCCATGTTACTT
CACCTGCTGTCGCATATGTTTGGGGGGAAATTGCTCGGA
TATCTTCATTTGGTTGCTCGTATATCTTCATTTGGTTTC
AATCGACGGCGTGTGTCAGCTGTGGAAAGTTGATGGTTG
GTACCCGGTGACGAATCGCAGGTTAATTAGTGGAAGACG
CGTGAAAAAAAAAAAAAAACCGAATTC

The DNA sequence represented by the formula [I] includes a DNA sequence wherein at least one base is substituted by degeneracy of codon.

Also provided is a DNA sequence complementary to the DNA sequence represented by the formula [I].

In addition, in accordance with the present invention, there is provided an antigenic protein specific to *Toxoplasma gondii* comprising at least a portion of an amino acid sequence represented by the following sequence [II]:

Ala Tyr Ala Ala Glu Gly Gly Asp Asn Gln
Ser Ser Ala Val Ser Asp Arg Ala Ser Leu
Phe Gly Leu Leu Ser Gly Gly Thr Gly Gln
Gly Leu Gly Ile Gly Glu Ser Val Asp Leu
Glu Met Met Gly Asn Thr Tyr Arg Val Glu
Arg Pro Thr Gly Asn Pro Asp Leu Leu Lys
Ile Ala Ile Lys Ala Ser Asp Gly Ser Tyr
Ser Glu Val Gly Asn Val Asn Val Glu Glu
Val Ile Asp Thr Met Lys Ser Met Gln Arg
Asp Glu Asp Ile Phe Leu Arg Ala Leu Asn
Lys Gly Glu Thr Val Glu Glu Ala Ile Glu
Asp Val Ala Gln Ala Glu Gly Leu Asn Ser
Glu Gln Thr Leu Gln Leu Glu Asp Ala Val Ser Ala Val Ala Ser Val Val Gln Asp Glu
Met Lys Val Ile Asp Asp Val Gln Gln Leu
Glu Lys Asp Lys Gln Gln Leu Lys Asp Asp
Ile Gly Phe Leu Thr Gly Glu Arg Glu

Said protein is preferably produced by means of a recombinant DNA technology, but may also be produced by means of a peptide synthesis method.

Other objects and advantages of the present invention will be made apparent as the description progresses.

## BRIEF DESCRIPTION OF THE DRAWINGS

Fig. 1 shows a *T. gondii*-specific novel DNA sequence of the present invention and amino acid sequence of a protein encoded by the DNA sequence.

Fig. 2 shows the restriction enzyme digestion map and the direction of sequencing used for the determination of a base sequence of cDNA.

## DETAILED DESCRIPTION OF THE INVENTION

A large scale preparation of *T. gondii* tachyzoites may be carried out by inoculating a virulent strain of *T. gondii*, RH strain, into the peritoneal cavity of mice and growing the tachyzoites in the cavity. The tachyzoites thus propagated were recovered together with peritoneal fluids, washed well with physiological saline and centrifuged in order to purify them. The resultant purified tachyzoites were cryopreserved in liquid nitrogen.

Extraction of mRNA from the frozen tachyzoites may be achieved by a combination of commonly used guanidinium thiocyanate method, hot phenol method and oligo(dT)- cellulose-aided column or batch process. For example, firstly, the frozen tachyzoites are mixed with iced particles of a solution of 4 M guanidinium thiocyanate prepared using liquid nitrogen in advance and ground thoroughly under the frozen conditions. After heating the ground powder, resultant suspension is thoroughly mixed with a hot phenol solution. Water layer is recovered from the mixture, subjected to a phenol/chloroform extraction process and then treated with proteinase. After repeating the phenol/chloroform extraction process, total RNA preparation is obtained by an ethanol precipitation method. Then, poly(A)$^+$ RNA (mRNA) is isolated by means of an oligo-dT cellulose column chromatography.

Synthesis of cDNA may be achieved by techniques commonly used in the field of recombinant DNA technology, using thus recovered mRNA as the template. For example, by using poly(A)$^+$ RNA as a template and oligo-d$T_{12-18}$ as a primer, first-strand cDNA synthesis is catalyzed by reverse transcriptase in the presence of dNTP (N represents A, G, C or T). RNase H nicks the RNA strand of the RNA-cDNA duplex formed in the first cDNA synthesis, and DNA polymerase I replaces the RNA with DNA.

Preferably, the cDNA thus obtained is cloned into a phage vector which has an ability to bring on the expression of a protein encoded by the base sequence of the cDNA. For example, the synthesized cDNA is firstly purified by means of a gel filtration after treating it with Klenow enzyme in order to blunt-end the double-stranded cDNA. To this is attached *Eco* RI adapters in the presence of ATP, T4 DNA ligase and T4 polynucleotide kinase. A recombinant phage is then obtained by ligating the adapter-attached cDNA into *Eco* RI arms of λgt 11 phage vector, followed by *in vitro* packaging. The above procedure can also be performed using commercial assay kits.

For the purpose of detecting a clone having a cDNA which encodes the antigenic protein of interest, cDNA library is prepared by plaquing the recombinant phage on *E. coli* Y1090 cells on an agar medium. The plaques are overlaied with nitrocellulose filters and then the plaque-absorbed filters are screened for the clone using an antiserum preparation obtained from a patient infected upon by *T. gondii.*

Detection of immune complexes may be carried out by an immunological means using an anti-human IgG antibody conjugated with horse radish peroxidase, hydrogen peroxide ($H_2O_2$) and a solution of 4-chloro-1-naphthol as a coloring substance.

Positive clones thus obtained are then subjected to the epitope selection method (*Seikagaku*, vol. 59, No. 7, p. 457, 1987) for the selection of clones encoding a protein having a molecular weight of about 27,000 daltons in the *in vitro* translation products of *T. gondii* total cellular RNA. In this process, antibodies specific for the positive clones are obtained from serum of an infected patient and screening of in vitro translation products of total *T. gondii* RNA is carried out using these antibodies. For example, proteins produced by the positive clones are allowed to react with serum from an infected patient to recover antibodies specific for the proteins. Immunoprecipitation reaction is carried out by reacting each of the antibodies obtained from each positive clone with *in vitro* translation products of the total *T. gondii* RNA one by one. Proteins precipitated by this reaction are then analyzed by electrophoresis in order to find a protein having a molecular weight of about 27,000 daltons. In this manner, a clone which produces a protein having an ability to undergo a specific immune reaction with an antibody that can recognize only a protein having a molecular weight of about 27,000 daltons is screened.

DNA sequence of a clone thus selected may be analyzed by the Maxam-Gilbert technique (*Methods in Enzymology*, vol. 65, Part 1, p. 497, 1980), but is preferably determined by the chain termination method (*Science*, vol. 214, p. 1205, 1981). For example, a cDNA insert is isolated from the selected clone by *Eco* RI digestion and subcloned into plasmid pTZ 18R, which was digested with *Eco* RI and treated with a phosphatase in advance, in the presence of T4 DNA ligase. The subcloned plasmid is purified by transforming it into *E. coli* JM 101 competent cells and isolating a transformant which forms a white colony on an agar medium in the presence of X-gal.

Thereafter, the cDNA insert may be digested into more short fragments using proper restriction enzymes and subjected to additional subcloning, in order to make the base sequence determination easy. For example, the cDNA is digested with a proper restriction enzyme, such as *Hind* III, *Eco* RV, *Kpn* I or *Acc* II, and subcloned into a corresponding site of a plasmid , such as pTZ 18U, pTZ 19U or pUC 119. Adjustment of the length of the cDNA insert for use in the determination of base sequence may also be carried out by means of preparing a deletion mutant using *Bal* 31 nuclease.

A base sequence is then determined by the dideoxy chain termination method in the presence of SequenaseTM, using these cloned plasmids. The following sequence [I] exemplifies the DNA sequence thus determined.

| | | | | | | | | | | |
|---|---|---|---|---|---|---|---|---|---|---|
| | | | | | | | | | C | 1 |
| GCT | TAC | GCT | GCC | GAA | GGC | GGC | GAC | AAC | CAG | 31 |
| TCG | AGC | GCC | GTC | TCA | GAT | CGG | GCG | TCT | CTC | 61 |
| TTT | GGT | TTG | CTG | AGT | GGA | GGG | ACA | GGG | CAG | 91 |
| GGA | TTA | GGA | ATC | GGA | GAA | TCT | GTA | GAT | TTG | 121 |
| GAG | ATG | ATG | GGG | AAC | ACG | TAT | CGT | GTG | GAG | 151 |
| AGA | CCC | ACA | GGC | AAC | CCG | GAC | TTG | CTC | AAG | 181 |
| ATC | GCC | ATT | AAA | GCT | TCA | GAT | GGA | TCG | TAC | 211 |
| AGC | GAA | GTC | GGC | AAT | GTT | AAC | GTG | GAG | GAG | 241 |
| GTG | ATT | GAT | ACT | ATG | AAA | AGC | ATG | CAG | AGG | 271 |
| GAC | GAG | GAC | ATT | TTC | CTT | CGT | GCG | TTG | AAC | 301 |
| AAA | GGC | GAA | ACA | GTA | GAG | GAA | GCG | ATC | GAA | 331 |

```
GAC GTG GCT CAA GCA GAA GGG CTT AAT TCG 361

GAG CAA ACC CTG CAA CTG GAA GAT GCA GTG 391

AGC GCG GTG GCG TCT GTT GTT CAA GAC GAG 421

ATG AAG GTG ATC GAC GAT GTG CAG CAG CTT 451

GAA AAG GAC AAA CAA CAG CTT AAG GAT GAC 481

ATT GGG TTC CTA ACA GGA GAG AGA GAG TAA 511

TTGGAGGTAGCTTTGTTGTGATCGTACCCCATGTTACTT 550

CACCTGCTGTCGCATATGTTTGGGGGAAATTGCTCGGA 599

TATCTTCATTTGGTTGCTCGTATATCTTCATTTGGTTTC 628

AATCGACGGCGTGTGTCAGCTGTGGAAAGTTGATGGTTG 667

GTACCCGGTGACGAATCGCAGGTTAATTAGTGGAAGACG 706

CGTGAAAAAAAAAAAAAAACCGAATTC             733.
```

The DNA sequence of the present invention may be at least a portion of the DNA sequence shown above as the sequence [I], or a DNA sequence which contains the DNA sequence [I] as a part.

The DNA sequence of the present invention may be synthesized either by using a template mRNA as described above or by means of organic synthetic chemistry.

In the field of recombinant DNA technology, in general, at least one base in the DNA sequence of a gene can be replaced with a different base without altering the amino acid sequence of protein produced from the gene, in accordance with the degeneracy of codon. Therefore, the DNA sequence of the present invention may comprise a different base sequence which is changed by a base substitution originated from the degeneracy of codon. In such a case, amino acid sequence deduced from the altered DNA sequence by the base substitution will coincide with the amino acid sequence which is shown later as sequence [II].

According to the present invention, a DNA sequence which is complementary to the DNA sequence represented by the sequence [I] may also be provided. In that case, the complementary DNA sequence may be complementary to at least a portion of the DNA sequence shown above as the sequence [I]. A DNA sequence which contains the complementary DNA sequence as a part may also be provided.

According to the present invention, the DNA of the sequence [I] and its complementary DNA may form a double-stranded DNA by their complementary linkage, or may be in the state of a complementary single-stranded DNA.

Amino acid sequence of the antigenic protein deduced from the DNA sequence represented by the sequence [I] is shown as the following sequence [II]:

Ala Tyr Ala Ala Glu Gly Gly Asp Asn Gln
Ser Ser Ala Val Ser Asp Arg Ala Ser Leu
Phe Gly Leu Leu Ser Gly Gly Thr Gly Gln
Gly Leu Gly Ile Gly Glu Ser Val Asp Leu
Glu Met Met Gly Asn Thr Tyr Arg Val Glu
Arg Pro Thr Gly Asn Pro Asp Leu Leu Lys
Ile Ala Ile Lys Ala Ser Asp Gly Ser Tyr
Ser Glu Val Gly Asn Val Asn Val Glu Glu
Val Ile Asp Thr Met Lys Ser Met Gln Arg
Asp Glu Asp Ile Phe Leu Arg Ala Leu Asn
Lys Gly Glu Thr Val Glu Glu Ala Ile Glu

Asp Val Ala Gln Ala Glu Gly Leu Asn Ser
Glu Gln Thr Leu Gln Leu Glu Asp Ala Val
Ser Ala Val Ala Ser Val Val Gln Asp Glu
Met Lys Val Ile Asp Asp Val Gln Gln Leu
Glu Lys Asp Lys Gln Gln Leu Lys Asp Asp
Ile Gly Phe Leu Thr Gly Glu Arg Glu

The *T. gondii*-specific protein of the present invention may consist of part or whole amino acid sequence expressed as the expression [II] or comprise the amino acid sequence [II] as a part of the protein.

It is possible to induce a spontaneous or an artificial mutation at a site of DNA structure of a gene and, as a result, a corresponding site of peptide structure derived from the mutated DNA structure without altering the main activity of the protein produced from the gene. In consequence, it is possible that the *T. gondii*-specific protein of the present invention has a structure of mutated amino acid sequence which is completely homologous to the above-mentioned amino acid sequence.

The *T. gondii*-specific protein containing substantially no other *T. gondii*-originated proteins may be obtained by performing the following order of steps: 1) isolation of a replicable recombinant DNA by means of ligation of the DNA sequence of the present invention into a replicable expression vector, 2) formation of transformants by transforming a microorganism or a cell with the replicable recombinant DNA, 3) selection of the transformant from parent cells of said microorganism or cell, 4) production of the *T. gondii*-specific protein in the cells of the selected transformant by culturing said transformant under certain conditions to induce expression of said DNA sequence in the cells, and 5) isolation of the produced protein from the cultured transformed cells or the culture media.

Such a process for obtaining the *T. gondii*-specific protein from a recombinant host cell is applicable to a large scale manufacture of said protein and, since said protein contains substantially no other *T. gondii*-originated proteins, an antibody specific for *T. gondii*-specific protein may be prepared using said protein.

## EXAMPLES

Examples of the present invention are given below by way of illustration, and not by way of limitation. Unless otherwise stated, abbreviations, code addresses and the like used in the following examples are those commonly used in the field concerned.

### Example 1

Large scale preparation of *Toxoplasma gondii* tachyzoites.

Large scale preparation of *Toxoplasma gondii* (to be referred to as *T. gondii* hereinafter) tachyzoites was carried out by infecting mice with the tachyzoites by means of the commonly used subculture method and recovering the propagated tachyzoites from the peritoneal cavities of mice. A virulent strain of *T. gondii*, RH strain, was kindly provided by Asst. Prof. Kiyoko Kamei at Parasitology, School of Medicine, Teikyo University, and inoculated into the peritoneal cavities of mice. Subculture of the RH strain was carried out at an interval of 4 days by collecting peritoneal fluids of mice, diluting the fluids to 1/10 to 1/20 with physiological saline and inoculating the diluted fluids into the peritoneal cavities of other fresh mice. In this manner, a total of 70 ddY mice were infected with the RH strain. These mice in their 4th day of the infection were killed by drawing blood, and the tachyzoites in the peritoneal cavities were washed and recovered in about 3 ml of physiological saline. The washing process was repeated 2 to 3 times. The peritoneal fluids thus collected were centrifuged at 1,200 x g for 8 minutes and the resultant pellet was suspended in 0.5 to 1 ml of physiological saline. The suspension containing tachyzoites was centrifuged again at 1,200 x g for 3 minutes and the final pellet, after removing the supernatant fluid, was frozen in liquid nitrogen and stored until required.

### Example 2

Extraction of mRNA from *T. gondii* tachyzoites.

1) Extraction of total RNA

Extraction of total RNA was carried out in accordance with the guanidinium-hot phenol method as follows. A 600 mg portion of the frozen pellet of *T. gondii* tachyzoites was mixed with 4.0 ml of a 4 M guanidinium thiocyanate solution containing 50 mM Tris-HCl (pH 7.6), 10 mM EDTA, 2% Sarkosyl and 1% β-mercaptoethanol, which has been made into ice particles in advance by dropping the solution into liquid nitrogen, and ground thoroughly under the frozen conditions using a grinder (Freezer Mill Type 6700, manufactured by Spex Industries Inc.). The powdered preparation was transferred into a test tube and incubated at 60° C to obtain a viscous solution. The viscosity was reduced by repeatedly passing the solution through a 18G injection needle 10 times in order to cut DNA molecules in the solution.

Next, the resultant solution was mixed thoroughly with 2.5 ml of phenol which has been heated to 60° C. A 1.2 ml portion of a 0.1 M sodium acetate solution (pH 5.2) and 2.5 ml of chloroform were added to the mixture and mixed vigorously at 60° C for 15 minutes. The mixture was centrifuged at 14,000 x g for 5 minutes at 4° C to recover water layer. The water layer was extracted once with phenol/chloroform (1:1) system and then twice with chloroform. After subjecting the extract to an ethanol precipitation process, the resultant RNA pellet was suspended in 0.1 M Tris-HCl buffer (pH 7.4) containing 50 mM NaCl, 10 mM EDTA and 0.2% SDS, mixed with proteinase K (final concentration, 200 µg/ml) and then incubated at 37° C for 60 minutes. After finishing the reaction, by heating at 60° C, the mixture was extracted twice with phenol and then twice with chloroform. Thereafter, total RNA was recovered by ethanol precipitation and dissolved in 300 µl of sterile distilled water. By performing these steps, 3 mg of total RNA was extracted.

2) Extraction of mRNA

Extraction of poly(A)$^+$ RNA from total cellular RNA was carried out using a commercial poly(A)$^+$ RNA isolation kit (manufactured by Funakoshi Pharmaceutical Co., Ltd.) in accordance with the manufacturer's instructions as follows. Oligo(dT)-cellulose was swollen in an equilibration solution (20 mM Tris-HCl (pH 7.6), 1 mM EDTA and 0.1% SDS) containing 0.5 M NaCl in order to prepare a column having 0.2 ml capacity. The column was washed successively with distilled water which was previously treated with 0.1% diethylpyrocarbonate and autoclave (DEPC distilled water), 0.1 M NaOH solution containing 5 mM EDTA, DEPC distilled water and the 0.5 M NaCl-containing equilibration solution in that order. A 1 mg portion of total RNA obtained in 1) of Example 2 was dissolved in DEPC distilled water (final volume, 200 µl) and incubated at 65° C for 5 minutes in order to perform heat denaturation. The solution was cooled in an ice bath immediately and mixed with 200 µl of the double-strength equilibration solution.

The resultant solution was applied to the column, washed with 2.5 ml of the equilibration solution containing 0.5 M NaCl and 1 ml of the equilibration solution containing 0.1 M NaCl and then the poly(A)$^+$ RNA retained by the column was eluted with 0.6 ml of a NaCl-free elution solution containing 10 mM Tris-HCl (pH 7.5), 1 mM EDTA and 0.05% SDS. The eluted fractions were pooled and applied again to the heating process at 65° C for 5 minutes and the same column chromatography. A 2 µl portion of sample was collected from each fraction and subjected to 0.8% agarose gel electrophoresis in order to detect fractions containing poly(A)$^+$ RNA, and the positive fractions (total volume, about 450 µl) were pooled. After adding 40 µl of 3 M sodium acetate solution, the pooled fraction was subjected to ethanol precipitation process, kept at -80° C for 15 minutes and then centrifuged at 14,000 x g for 10 minutes to recover poly(A)$^+$ RNA. By performing these steps, a total of 15 µg of poly(A)$^+$ RNA was recovered from 1 mg of total cellular RNA.

Example 3

Construction of cDNA library.

Construction of cDNA library from the poly(A)$^+$ RNA preparation obtained in Example 2 was carried out using a commercial cDNA synthesis kit (manufactured by Pharmacia LKB Biotechnology AB, code No. 27-9260-01) in accordance with the manufacturer's instructions as follows. A 5 µg portion of poly(A)$^+$ RNA (mRNA) obtained in Example 2 was dissolved in the DEPC distilled water (final volume, 20 µl). The solution was incubated at 65°C for 10 minutes and then cooled in an ice bath. To this was added 11 µl of the first reaction solution of the kit, which contained Tris-HCl, dNTP (N represents A, G, C or T), oligo-dT primers and Moloney mouse leukemia virus reverse transcriptase, 1 µl of DTT solution of the kit and 1 µCi of [$^{32}$P] dCTP. Synthesis of single-stranded cDNA was achieved by incubating this mixture at 37°C for 60 minutes.

The solution of single-stranded cDNA thus obtained was mixed with 68 µl of the second reaction solution of the kit, which contained dNTP, *E. coli* RNase H and DNA polymerase I, and 50 µCi of [$^{32}$P] dCTP and incubated at 12°C for 60 minutes, followed by the second incubation at 22°C for 60 minutes. The reaction mixture was then mixed with 1 µl of Klenow enzyme solution of the kit and incubated at 37°C for 30 minutes. Total amount of cDNA thus synthesized was estimated to be 1.6 µg by assaying incorporated radioactivity into the cDNA. The Klenow enzyme reaction was stopped by adding 100 µl of a phenol/chloroform solution, and the reaction mixture was subjected to the cDNA extraction process as follows. Water layer of the phenol/chloroform-added mixture was applied to a Sephacryl S-200 spun column chromatography and eluted with 66 mM Tris-HCl buffer (pH 7.6) containing 1 mM spermidine, 10 mM $MgCl_2$, 15 mM DTT and 0.2 mg/ml of BSA. The eluent as such was then used for the ligation of *Eco* RI adapters.

The eluent was mixed with 5 µl of *Eco* RI adapter solution of the kit, 1 µl of ATP solution of the kit and 3 µl of T4 DNA ligase solution of the kit, and incubated overnight at 12°C. The reaction mixture was incubated at 65°C for 10 minutes in order to inactivate the enzymes. After cooling in an ice bath, the reaction mixture was further mixed with 10 µl of ATP solution of the kit and 1 µl of T4 polynucleotide kinase of the kit and incubated at 37°C for 30 minutes. Thereafter, the reaction solution was mixed with 100 µl of the phenol/chloroform solution to extract cDNA, and the resultant water layer was applied again to the spun column chromatography. By performing these steps, 250 ng of cDNA molecules having *Eco* RI terminals were recovered in the eluent. After subjecting the eluent to ethanol precipitation process, the cDNA molecules were dissolved in sterile distilled water to a concentration of 10 ng/µl.

Cloning of cDNA into phage vector was carried out using a commercial DNA ligation kit (manufactured by Takara Shuzo Co., Ltd., code No. 6021) according to the manufacturer's instructions as follows. To 1 µl cDNA solution (10 ng cDNA) was added Tris-HCl (pH 7.6), $MGCl_2$ and NaCl to their final concentrations of 100 mM, 5 mM and 300 mM, respectively, and 250 ng of previously phosphatase-treated *Eco* RI arms of λgt 11 phage. The solution thus prepared (4.0 µl) was mixed with 4.0 µl of B solution of the ligation kit and incubated at 26°C for 10 minutes.

The recombinant phage DNA thus obtained was then subjected to *in vitro* packaging using a commercial Gigapack plus kit (manufactured by Stratagene Cloning Systems). That is, 0.1 µg of the recombinant phage DNA was added to the packaging solution of the kit, mixed gently and incubated at room temperature for 2 hours. The resultant solution was mixed with 500 µl of the dilution solution of the kit and 20 µl of chloroform and stored at 4°C.

## Example 4

### Immunological screening of λgt 11 cDNA library using antibody

1) Formation of phage plaques.

*E. coli* Y 1090 was cultured overnight using LB medium (pH 7.5; Bacto Tryptone, 10 g/l; Bacto-Yeast Extract, 5 g/l; and NaCl, 10 g/l) which has been supplemented with 50 µg/ml of ampicillin and 2 g/l of maltose. A 0.2 ml portion of the cultured broth was mixed with 0.1 ml of the recombinant phage (5 ~ 10 x $10^3$ p.f.u.) which had been prepared according to Example 3 and incubated at 37°C for 15 minutes in order to complete transfection. The incubated solution was mixed with 2.5 ml of LB medium supplemented with 0.65% agarose and the mixture was poured and solidified on a plate containing LB-agar medium. After incubating the plate at 42°C for 4 hours, a nitrocellulose filter which had been soaked with 10 mM isopropyl-$\beta$-D-thiogaractopyranoside (IPTG) was put on the overlay agar medium and the plate was incubated again at 37°C for 2 hours. Phage plaques thus formed were replicated onto the filter. As the result, 3 x $10^4$ plaques were obtained.

(2) Screening using antibody

The replica filters thus obtained were washed at room temperature for 5 minutes using TBS solution (pH 7.5) supplemented with 50 mM Tris-HCl and 150 mM NaCl and then incubated at room temperature for 1 hour in TBS solution supplemented with 0.5% skimmed milk. The filters were incubated overnight at room temperature in TBS solution containing 0.5% skimmed milk, 10% Y 1090 lysate and 1% anti-*T. gondii* antiserum as the primary antibody. The filters were washed three times (5 min for each) at room temperature with 100 mM phosphate buffer (pH 7.0) containing 0.05% Tween 20 in order to remove unreacted antibody. The filters were washed again with TBS solution for 10 minutes in the same manner. The washed filters were incubated at room temperature for 1 hour in TBS solution containing 2.5% of gelatin and 0.1% of a peroxidase-labelled anti-human IgG antibody (manufactured by Medical & Biological Laboratories Co., Ltd.) as the secondary antibody.

The filters were washed three times (5 min for each) at room temperature with 100 mM phosphate buffer (pH 7.0) containing 0.05% Tween 20 in order to remove unreacted antibody. The filters were washed again with TBS solution for 10 minutes and then soaked in a coloring solution in order to screen positive clones from the filters. The coloring solution was prepared by mixing a portion of 4-chloro-1-naphthol solution dissolved in methanol as a color reagent (3 mg/ml) with 1/400 volume of 30% hydrogen peroxide ($H_2O_2$) as a substrate and 5 volumes of TBS solution. Identification of positive clones were carried out by repeating these steps. As the results, 26 positive clones were obtained based on the coloring reaction.

## Example 5

### Screening of positive clones

Screening of positive clone was carried out as follows in accordance with the epitope selection method (*Seikagaku,* vol. 59, No. 7, p. 457, 1987).

For this purpose, the following four solutions were prepared:

(1) skimmed milk solution consisting of 0.5% (w/v) skimmed milk, 20 mM Tris-HCl (pH 8.0) and 100 mM NaCl,

(2) elution solution I consisting of 5 mM glycine-HCl (pH 2.3), 0.15 M NaCl and 0.1 mg/ml BSA,

(3) 1 M Tris-HCl solution, and

(4) washing solution consisting of 0.05% Tween 20 dissolved in the skimmed milk solution.

Eight clones were selected at random from the 26 positive clones identified in Example 4, and each of the corresponding plaques of the 8 positive clones was collected from the soft agar medium and suspended in 1 ml of SM medium consisting of 50 mM of Tris-HCl (pH 7.5), 5.8 g/l of NaCl, 2 g/l of $MgSO_4 \cdot 7H_2O$ and 0.1 g/l of gelatine. A 1 µl portion of the phage suspension was mixed with 0.2 ml of a broth of *E. coli* Y1090 cultured overnight in SM medium, poured onto a plate medium and incubated at 42°C for 4 hours. A nitrocellulose filter which had been soaked with IPTG was put on the plate medium and the plate was incubated again for 2 hours. Thereafter, the filter was removed from the plate and incubated with shaking in the skimmed milk solution at room temperature for 1 hour.

Next, 500 µl of the antiserum preparation obtained from a patient of toxoplasmosis was diluted with 25 ml of the skimmed milk solution supplemented with 0.02% of sodium azide. The filter was soaked in this antiserum solution, incubated overnight at room temperature and then washed with 5 ml of the washing solution three times (10 minutes for each). Antibodies adsorbed to the filter were eluted out by shaking the filter in 2 ml of the elution solution twice (1 minutes for each) and further shaking it in 1 ml of the same solution for 1 minutes. The eluent (total 5 ml) was immediately cooled in an ice bath and neutralized with 0.3 ml of the 1 M Tris-HCl solution. The neutralized eluent was then mixed with 100 µl of 25% skimmed milk solution and 10 μl of 10 % sodium azide solution in that order and used as the antibody solution. These steps were repeated for each of the 8 clones.

Screening of *in vitro* translation products of total RNA was carried out using these 8 antibody solutions. The *in vitro* translation was carried out using 2 μg of total *T. gondii* cellular RNA obtained according to Example 2 and a commercial rabbit reticulocyte lysate system (manufactured by Amersham) in accordance with the manufacturer's instructions in the presence of 10 μCi [$^{35}$S] methionine. A 10 μl portion of the translation products thus obtained was mixed with 100 μl of one of the 8 antibody solutions and incubated at 4° C for 16 hours. The resultant reaction solution was mixed with 5 μl of 40% (v/v) cell suspension of *Staphylococcus aureus* strain Cowan 1 and incubated at room temperature for 30 minutes. The incubated mixture was centrifuged at 10,000 x g for 2 minutes and the resultant pellet, after discarding the supernatant fluid, was washed four times with 50 mM Tris-HCl buffer (pH 7.6) containing 150 mM NaCl, 5 mM EDTA and 0.02% $NaN_3$.

Then the pellet thus washed was suspended in 150 mM Tris-HCl buffer (pH 6.8) containing 4% SDS, 10% β-mercaptoethanol, 20% glycerol and 0.02% bromophenol blue and incubated at 100° C for 8 minutes. After centrifugation of the incubated suspension at 10,000 x g for 2 minutes, proteins in the resultant supernatant fluid was isolated by sodium dodecyl sulphate-polyacrylaminde gel electrophoresis (SDS-PAGE) and the isolated proteins were analyzed by an autoradiography. Translated proteins thus obtained using the 8 antibody solutions were checked on the SDS-PAGE for the presence of a band specific to about 27,000 daltons of molecular weight, and positive clones which corresponded to the proteins showing the specific band were selected for further use.

## Example 6

### Subcloning of positive clone

The phage plaque (λTg 18) identified in Example 5 was collected together with the agar medium using a Pasteur pipette and suspended in 1 ml of SM medium to make a phage suspension. A 200 μl portion of the phage suspension was mixed with a portion of a broth of *E. coli* Y1088 cultured overnight in SM medium and incubated at 37° C for 6 hours in order to complete transfection. The resultant mixture was mixed with 0.1 ml of chloroform, incubated at 37° C for 10 minutes and then centrifuged at 6,000 x g for 10 minutes.

The supernatant fluid thus obtained was mixed with DNase I and RNase A to their final concentration of 50 μg/ml and incubated at 37° C for 30 minutes. After the incubation, the resultant solution was mixed with 0.6 volume of 20% polyethylene glycol solution containing 2.5 M NaCl and kept overnight at 4° C. The resultant solution was centrifuged at 6,000 rpm for 15 minutes, the pellet thus obtained was suspended in 500 μl of 10 mM Tris-HCl buffer (pH 7.5) containing 5 mM EDTA and 0.5% SDS and the suspension was incubated at 37° C for 20 minutes. Isolation of the phage DNA was carried out in accordance with the commonly used procedure, that is, phenol extraction, phenol/chloroform extraction and ethanol precipitation in that order.

The pellet obtained by the ethanol precipitation was washed with ethanol, air-dried and then dissolved in 200 μl of a TE solution which consisted of 10 mM Tris-HCl (pH 7.5) and 1 mM EDTA. The resultant solution was mixed with ammonium acetate to its final concentration of 2 M, and the ethanol precipitation process was repeated. The pellet was finally dissolved in the TE solution such that the final concentration of the phage DNA became 200 μg/ml. To 5 μl portion of this DNA preparation was added 1 μl of 10 U/μl *Eco* RI enzyme solution and 10 μl of a buffer solution for *Eco* RI use, and the resultant mixture (total volume, 16 μl) was incubated at 37° C for 20 minutes in order to digest the DNA. The *Eco* RI digests were then mixed with 32 μl of 90% ethanol containing 0.1 M NaCl and kept at -80° C for 10 minutes to complete the ethanol precipitation process, and the resultant pellet was air-dried.

Next, 1 μg of the DNA preparation thus obtained (50 ng as the cDNA insert) was used for the subcloning of the cDNA into plasmid pTZ 18R. The subcloning was carried out using a commercial DNA ligation kit (manufactured by Takara Shuzo Co., Ltd.) as follows. The DNA preparation (1 μg) was mixed with 1 μl of sterile distilled water and 0.5 μl of the plasmid vector pTZ 18R which was previously treated with *Eco* RI and phosphatase in this order (50 ng), kept at room temperature for 5 minutes and then mixed again thoroughly. To this was added 0.5 μl of 0.4 M Tris-HCl buffer (pH 7.5) containing 20 mM $MGCl_2$ and

16 µl of the A solution of the DNA ligation kit. After mixing thoroughly, the mixture was mixed further with 2 µl of the B solution of the kit and incubated at 16° C for 30 minutes.

Then, 3 µl portion of the recombinant plasmid solution thus obtained was added to 100 µl of a suspension of *E. coli* JM101 competent cells which have been prepared in accordance with a commonly used method (*Molecular Cloning, A Laboratory Manual,* Cold Spring Harbor Laboratory, p. 250, 1982). After mixing in an ice bath, the mixture was heated at 44° C for 1 minute and again kept for 1 to 2 minutes in an ice bath. The resultant mixture was further mixed with 100 µl of LB medium, 20 µl of 100 mM IPTG and 40 µl of 1% X-gal, poured onto an ampicillin-added plate medium and then incubated at 37° C overnight.

A white colony among colonies formed on the plate medium was isolated as the transformant and inoculated into 3 ml of LB medium and cultured with shaking at 37° C for a whole day and night. The cultured broth was centrifuged at 1,000 x g for 1 minute, and the resulting pellet was suspended in 100 µl of a glucose-lysozyme solution which consisted of 50 mM glucose, 10 mM EDTA, 25 mM Tris-HCl (pH 8.0) and 4 mg/ml lysozyme. After keeping at room temperature for 5 minutes, the suspension was mixed gently with 200 µl of an alkali solution which consisted of 0.2 N NaOH and 1% SDS and then cooled in an ice bath for 5 minutes. The resultant lysate was mixed thoroughly with 150 µl of 5 M potassium acetate solution, cooled in an ice bath for 5 minutes and then centrifuged at 12,000 rpm for 5 minutes at 4° C. Plasmids in the supernatant fluid were extracted by a phenol/chloroform extraction procedure and precipitated with ethanol, and the resulting pellet of plasmids was air-dried.

The dried pellet was then dissolved in 50 µl of the TE solution, and the plasmid solution was mixed with 0.5 µl of 1 mg/ml solution of RNase A (DNase-free) and incubated at 37° C for 1 hour. The incubated solution was mixed with 1/3 volume of a solution consisting of 20% polyethylene glycol and 2.5 M NaCl and kept at 0° C for 1 hour. Thereafter, the solution was centrifuged at 12,000 rpm for 10 minutes, and the resulting pellet was washed with ethanol, air-dried and then dissolved in 50 µl of the TE solution.

The transformant, named *E. coli* JM101 (pTZ 18R, pTg18), having the recombinant plasmid obtained in this manner has been deposited by the present inventors on February 23, 1989, in Fermentation Research Institute, Agency of Industrial Science and Technology, and has been assigned the designation as FERM BP-2766 (transferred from P-10565).

## Example 7

### Subcloning of cDNA insert

In order to obtain a cDNA fragment having an appropriate length for use in the determination of base sequence, the plasmid cDNA insert obtained in Example 6 was further subcloned as follows.

1) Digestion of cDNA insert.

A 10 µg portion of the purified plasmid obtained in Example 6 was digested with *Eco* RI, and a fragment having about 750 bp thus produced was isolated on 0.7% agarose gel electrophoresis. The isolated fragment was divided into 5 samples (0.2 µg for each) and digested further with 5 groups of restriction enzymes as shown in Table 1. The digestion reaction was stopped by heating the reaction solution at 60° C for 15 minutes, after confirming completion of the digestion of each sample on 5% polyacrylamide gel electrophoresis.

Table 1

| Group | Restriction enzyme |
|---|---|
| 1 | *Hind* III |
| 2 | *Eco* RV |
| 3 | *Acc* II |
| 4 | *Eco* RV + *Hind* III |
| 5 | *Kpn* I + *Hind* III |

2) Preparation of cloning vector.

Plasmids pTZ 18U, pTZ 19U and pUC 119 were digested with 8 groups of restriction enzymes as shown in Table 2 and used as cloning vectors for the fragments obtained in 1) of Example 7. That is, about 1 μg of each plasmid was digested with corresponding combinations of restriction enzymes as shown in Table 2, and the reaction was stopped by heating the reaction solution at 60° C for 15 minutes. The DNA fragments thus obtained were then recovered by the commonly used ethanol precipitation procedure, and the resulting pellet was air-dried and dissolved in 20 μl of the TE solution.

Table 2

| Group | Vector | Restriction enzyme |
|---|---|---|
| A | pTZ 18U | *Eco* RI + *Hind* III |
| B | pTZ 19U | *Eco* RI + *Hind* III |
| C | pTZ 18U | *Eco* RI + *Sma* I |
| D | pTZ 18U | *Kpn* I + *Hinc* II |
| E | pUC 119 | *Sma* I + *Eco* RI |
| F | pUC 119 | *Hind* III + *Hinc* II |
| G | pUC 119 | *Hind* III + *Kpn* I |
| H | pUC 119 | *Hind* III + *Eco* RI |

3) Ligation and transformation

A 2 μl portion (ca. 20 ng as DNA) of each one of the restriction enzyme digests of the cDNA insert obtained in 1) of Example 7 and 2 μl (ca. 100 ng as DNA) of each one of the restriction enzyme digests of the vector plasmids obtained in 2) of Example 7 were mixed according to the combinations as shown in Table 3. Each mixture thus prepared was mixed with 12 μl of the A solution of a commercial DNA ligation kit (manufactured by Takara Shuzo Co., Ltd.) and 3 μl of the B solution of the same kit and incubated at 16° C for 30 minutes. After the incubation, 1/2 volume of each of the reaction solution (9 μl) was added to 100 μl portion of a suspension of *E. coli* JM101 competent cells and incubated for 15 minutes in an ice bath.. The incubated cell suspension was immediately mixed with 400 μl of LB medium, and 1/10 volume of the mixture was inoculated, together with appropriate amounts of X-gal and IPTG, onto LB agar medium containing 100 μg/ml of ampicillin. After incubating the plate at 37° C overnight, a white colony formed on the plate medium was treated in the same manner as described in Example 6 to isolate and purify plasmid in the white colony. For the purpose of confirming if the plasmid thus obtained has a proper length of cDNA insert for use in the determination of base sequence, the cDNA insert in the plasmid was isolated by a restriction enzyme treatment and checked for its length.

Table 3

| Vector group | cDNA insert group |
|---|---|
| A | 1 |
| B | 1 |
| C | 2, 3 |
| D | 5 |
| E | 3 |
| F | 4 |
| G | 5 |
| H | 1 |

## Example 8

### Preparation of single-stranded DNA for base sequence determination

Each one of the white colonies which have been confirmed in Example 7 that their plasmids have the proper length of the cDNA insert was inoculated into 5 ml of 2 x TY medium (tryptone, 16 g/l; yeast extracts, 100 g/l; and NaCl, 5 g/l) supplemented with 50 µg/ml of ampicillin and cultured overnight at 37° C with shaking. One milliliter portion of the cultured broth was again inoculated into 50 ml of the same fresh medium (supplemented with 100 µg/ml of ampicillin) and cultured at 37° C for 30 minutes with shaking. Resultant broth of the second culturing was mixed with M13 K07 as a helper phage to an amount such that m.o.i. (multiplicity of infection) became about 20. After culturing the helper-added broth at 37° C for 30 minutes, kanamycin was added to its final concentration of 70 µg/ml and the culturing was continued overnight. After completion of the culturing, the cultured broth was centrifuged at 14,000 rpm for 15 minutes at 4° C to recover the supernatant fluid. The supernatant fluid was centrifuged again under the same conditions in order to remove the cells completely. To the supernatant fluid was added polyethylene glycol and ammonium acetate to their final concentrations of 4% and 0.7 M, respectively. After mixing gently and standing still for 30 minutes in an ice bath, the mixture was centrifuged at 14,000 rpm for 15 minutes at 4° C in order to precipitate phages. The phage pellet thus obtained was dissolved in 0.7 ml of TE solution, and single-stranded DNA in the solution was extracted using a phenol/chloroform extraction system and recovered by an ethanol precipitation process.

## Example 9

### Determination of cDNA base sequence

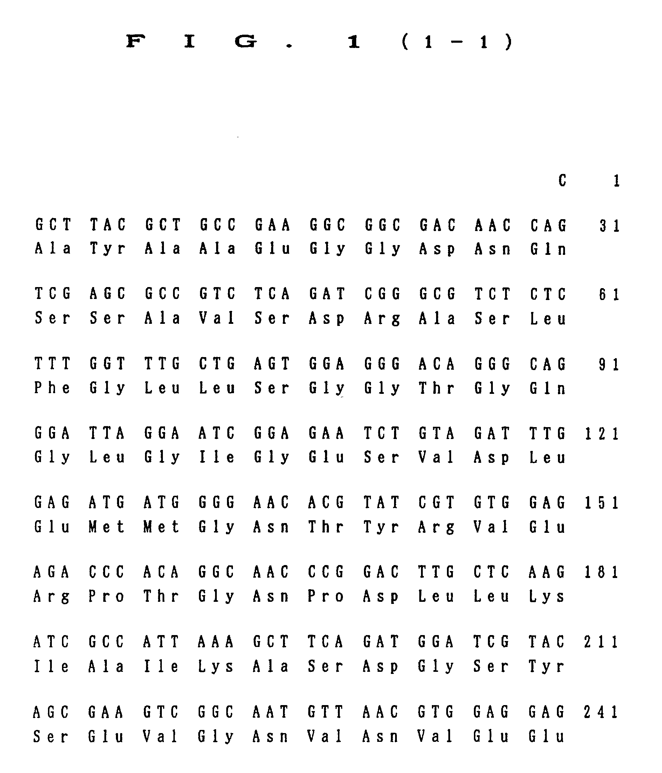

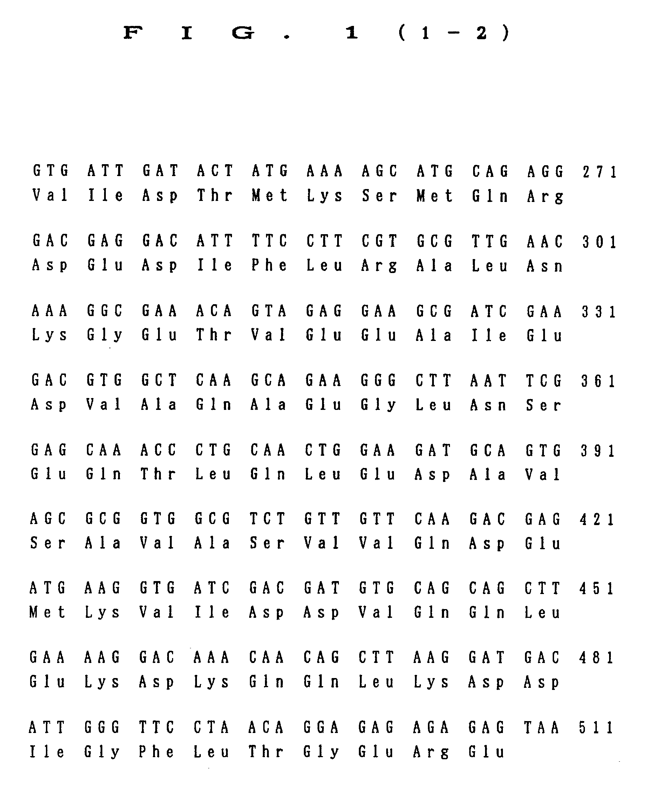

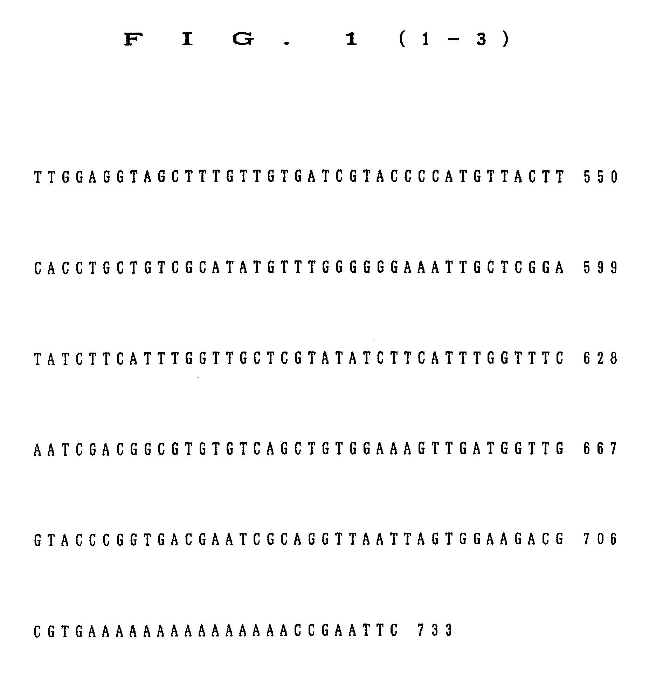

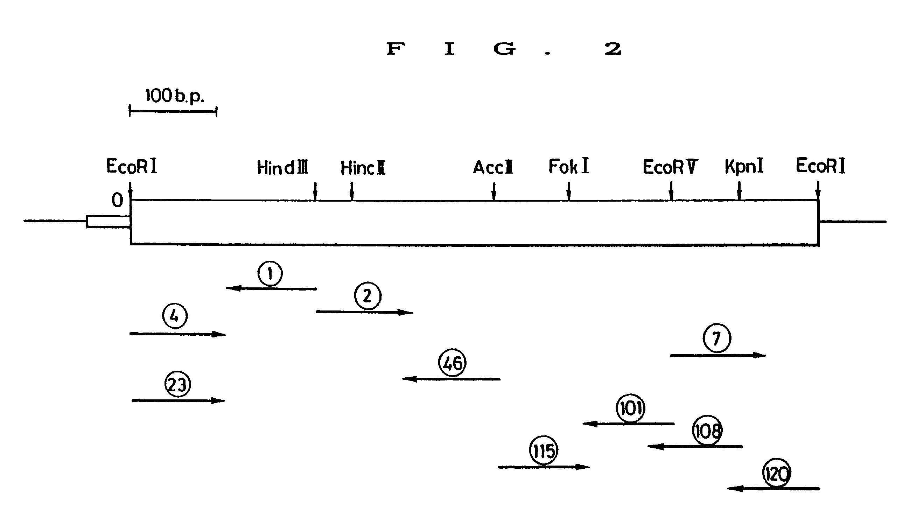

Base sequence of the cDNA obtained in Example 8 was determined by the dideoxy chain termination method using a commercial DNA polymerase preparation, SequenaseTM (manufactured by United States Biochemical Corp.). The sequencing strategy is shown in Fig. 2.

About 1 µg of a DNA preparation was dissolved in 7 µl of sterile distilled water, and the solution was mixed with 1 µl of M13 primer solution (0.5 p mol/1 µl) and 2 µl of TE solution which has been supplemented with 100 mM $MgCl_2$ and 250 mM NaCl. Annealing of the mixture was carried out by its serial incubation at 65° C for 10 minutes, at 42° C for 10 minutes and at room temperature for 10 minutes in that order.

Next, the annealed solution was mixed with 2.0 µl of one of 3 µM dNTP (N represents A, G or T) solutions, 2 µl of Sequenase™ solution (1.5 U/µl), 1 µl of 0.1 M DTT and 0.5 µl (5 µCi) of 400 Ci/m mol [$^{32}$P] dCTP, and the mixture was incubated at 37° C for 5 minutes. A 3.5 µl portion of the incubated isotope-labelled mixture was added to 3.5 µl of one of 8 µM ddNTP (N represents A, G, C or T) solutions and incubated at 37° C for 5 minutes. The reaction was stopped by adding 4 µl of a solution consisting of 95% deionized formamide, 25 mM EDTA, 0.1% bromophenol blue and 0.1% xylene cyanol and subsequently heating the mixture at 80° C for 2 minutes. The mixture was cooled rapidly in an ice bath and applied to a polyacrylamide gel electrophoresis and an autoradiography to determine the base sequence. Results of the sequencing and the amino acid sequence deduced from the base sequence are shown in Fig. 1. It was confirmed that the cloned DNA sequence according to the present invention was a region consisting of 733 bases having a poly(A) tail in its 3′ side and containing an open leading frame consisting of 522 bases.

Thus, it is apparent that there has been provided, in accordance with the present invention, a DNA sequence coding for an antigenic protein originated from and specific to *T. gondii* tachyzoites. Also provided is an amino acid sequence of a *T. gondii*-specific antigenic protein encoded by said DNA sequence.

According to the present invention, it is possible to manufacture by means of genetic engineering a *T. gondii*-specific antigen which contains substantially no other proteins originated from *T. gondii* tachyzoites. In consequence, it seems that a highly purified and specific antigen with greatly improved detection accuracy can be applied to an agent for use in the diagnosis of *T. gondii* infection, while any of the prior diagnoses of said infection has been depending on a crude antigen preparation. In addition, manufacturing of the *T. gondii*- specific antigen by means of genetic engineering renders possible complete elimination of a danger of infection by *T. gondii* upon the process workers.

Still more, it is possible to prepare a monoclonal antibody which is capable of undergoing a specific immune reaction with the *T. gondii*-specific antigen of the present invention making use of the amino acid sequence or the DNA sequence provided herein, and also to prepare a DNA or an RNA fragment complementary to said DNA sequence. Such preparations will contribute greatly to the development of an immunological diagnostic agent and a reagent for DNA diagnosis which can directly detect *T. gondii* in tissues or blood of an infected patient.

## Claims

1. A DNA sequence comprising at least a portion of a DNA sequence represented by the following sequence [I] which encodes a protein specific to *Toxoplasma gondii*:

GCT TAC GCT GCC GAA GGC GGC GAC AAC CAG
TCG AGC GCC GTC TCA GAT CGG GCG TCT CTC
TTT GGT TTG CTG AGT GGA GGG ACA GGG CAG
GGA TTA GGA ATC GGA GAA TCT GTA GAT TTG
GAG ATG ATG GGG AAC ACG TAT CGT GTG GAG
AGA CCC ACA GGC AAC CCG GAC TTG CTC AAG
ATC GCC ATT AAA GCT TCA GAT GGA TCG TAC
AGC GAA GTC GGC AAT GTT AAC GTG GAG GAG
GTG ATT GAT ACT ATG AAA AGC ATG CAG AGG
GAC GAG GAC ATT TTC CTT CGT GCG TTG AAC
AAA GGC GAA ACA GTA GAG GAA GCG ATC GAA
GAC GTG GCT CAA GCA GAA GGG CTT AAT TCG
GAG CAA ACC CTG CAA CTG GAA GAT GCA GTG
AGC GCG GTG GCG TCT GTT GTT CAA GAC GAG
ATG AAG GTG ATC GAC GAT GTG CAG CAG CTT
GAA AAG GAC AAA CAA CAG CTT AAG GAT GAC
ATT GGG TTC CTA ACA GGA GAG AGA GAG TAA
TTGGAGGTAGCTTTGTTGTGATCGTACCCCATGTTACTT
CACCTGCTGTCGCATATGTTTGGGGGGAAATTGCTCGGA
TATCTTCATTTGGTTGCTCGTATATCTTCATTTGGTTTC
AATCGACGGCGTGTGTCAGCTGTGGAAAGTTGATGGTTG
GTACCCGGTGACGAATCGCAGGTTAATTAGTGGAAGACG
CGTGAAAAAAAAAAAAAAACCGAATTC

2. The DNA sequence according to claim 1 having at least one base substituted by degeneracy of codon.

3. A DNA sequence complementary to said DNA sequence of claim 1.

4. A protein specific to *Toxoplasma gondii* wherein said protein has a molecular weight of 25,000 to 30,000 daltons and is capable of undergoing a specific immune reaction with an anti-*Toxoplasma gondii* antiserum.

5. A protein specific to *Toxoplasma gondii* comprising at least a portion of an amino acid sequence represented by the following sequence [II]:

Ala Ty Ala Ala Glu Gly Gly Asp Asn Gln
Ser Ser Ala Val Ser Asp Arg Ala Ser Leu
Phe Gly Leu Leu Ser Gly Gly Thr Gly Gln
Gly Leu Gly Ile Gly Glu Ser Val Asp Leu
Glu Met Met Gly Asn Thr Tyr Arg Val Glu
Arg Pro Thr Gly Asn Pro Asp Leu Leu Lys
Ile Ala Ile Lys Ala Ser Asp Gly Ser Tyr
Ser Glu Val Gly Asn Val Asn Val Glu Glu
Val Ile Asp Thr Met Lys Ser Met Gln Arg
Asp Glu Asp Ile Phe Leu Arg Ala Leu Asn
Lys Gly Glu Thr Val Glu Glu Ala Ile Glu
Asp Val Ala Gln Ala Glu Gly Leu Asn Ser
Glu Gln Thr Leu Gln Leu Glu Asp Ala Val
Ser Ala Val Ala Ser Val Val Gln Asp Glu
Met Lys Val Ile Asp Asp Val Gln Gln Leu
Glu Lys Asp Lys Gln Gln Leu Lys Asp Asp
Ile Gly Phe Leu Thr Gly Glu Arg Glu

6. The protein according to claim 5 wherein said protein is produced in a recombinant host cell.

7. The protein according to claim 6 wherein said recombinant host cell contains the DNA sequence of claim 1.

# FIG. 1 (1-1)

| | | | | | | | | | | |
|---|---|---|---|---|---|---|---|---|---|---|
| | | | | | | | | | C | 1 |
| GCT | TAC | GCT | GCC | GAA | GGC | GGC | GAC | AAC | CAG | 31 |
| Ala | Tyr | Ala | Ala | Glu | Gly | Gly | Asp | Asn | Gln | |
| TCG | AGC | GCC | GTC | TCA | GAT | CGG | GCG | TCT | CTC | 61 |
| Ser | Ser | Ala | Val | Ser | Asp | Arg | Ala | Ser | Leu | |
| TTT | GGT | TTG | CTG | AGT | GGA | GGG | ACA | GGG | CAG | 91 |
| Phe | Gly | Leu | Leu | Ser | Gly | Gly | Thr | Gly | Gln | |
| GGA | TTA | GGA | ATC | GGA | GAA | TCT | GTA | GAT | TTG | 121 |
| Gly | Leu | Gly | Ile | Gly | Glu | Ser | Val | Asp | Leu | |
| GAG | ATG | ATG | GGG | AAC | ACG | TAT | CGT | GTG | GAG | 151 |
| Glu | Met | Met | Gly | Asn | Thr | Tyr | Arg | Val | Glu | |
| AGA | CCC | ACA | GGC | AAC | CCG | GAC | TTG | CTC | AAG | 181 |
| Arg | Pro | Thr | Gly | Asn | Pro | Asp | Leu | Leu | Lys | |
| ATC | GCC | ATT | AAA | GCT | TCA | GAT | GGA | TCG | TAC | 211 |
| Ile | Ala | Ile | Lys | Ala | Ser | Asp | Gly | Ser | Tyr | |
| AGC | GAA | GTC | GGC | AAT | GTT | AAC | GTG | GAG | GAG | 241 |
| Ser | Glu | Val | Gly | Asn | Val | Asn | Val | Glu | Glu | |

# FIG. 1 (1-2)

| | | | | | | | | | | |
|---|---|---|---|---|---|---|---|---|---|---|
| GTG | ATT | GAT | ACT | ATG | AAA | AGC | ATG | CAG | AGG | 271 |
| Val | Ile | Asp | Thr | Met | Lys | Ser | Met | Gln | Arg | |
| GAC | GAG | GAC | ATT | TTC | CTT | CGT | GCG | TTG | AAC | 301 |
| Asp | Glu | Asp | Ile | Phe | Leu | Arg | Ala | Leu | Asn | |
| AAA | GGC | GAA | ACA | GTA | GAG | GAA | GCG | ATC | GAA | 331 |
| Lys | Gly | Glu | Thr | Val | Glu | Glu | Ala | Ile | Glu | |
| GAC | GTG | GCT | CAA | GCA | GAA | GGG | CTT | AAT | TCG | 361 |
| Asp | Val | Ala | Gln | Ala | Glu | Gly | Leu | Asn | Ser | |
| GAG | CAA | ACC | CTG | CAA | CTG | GAA | GAT | GCA | GTG | 391 |
| Glu | Gln | Thr | Leu | Gln | Leu | Glu | Asp | Ala | Val | |
| AGC | GCG | GTG | GCG | TCT | GTT | GTT | CAA | GAC | GAG | 421 |
| Ser | Ala | Val | Ala | Ser | Val | Val | Gln | Asp | Glu | |
| ATG | AAG | GTG | ATC | GAC | GAT | GTG | CAG | CAG | CTT | 451 |
| Met | Lys | Val | Ile | Asp | Asp | Val | Gln | Gln | Leu | |
| GAA | AAG | GAC | AAA | CAA | CAG | CTT | AAG | GAT | GAC | 481 |
| Glu | Lys | Asp | Lys | Gln | Gln | Leu | Lys | Asp | Asp | |
| ATT | GGG | TTC | CTA | ACA | GGA | GAG | AGA | GAG | TAA | 511 |
| Ile | Gly | Phe | Leu | Thr | Gly | Glu | Arg | Glu | | |

FIG. 1 (1-3)

TTGGAGGTAGCTTTGTTGTGATCGTACCCCATGTTACTT 550

CACCTGCTGTCGCATATGTTTGGGGGGAAATTGCTCGGA 599

TATCTTCATTTGGTTGCTCGTATATCTTCATTTGGTTTC 628

AATCGACGGCGTGTGTCAGCTGTGGAAAGTTGATGGTTG 667

GTACCCGGTGACGAATCGCAGGTTAATTAGTGGAAGACG 706

CGTGAAAAAAAAAAAAAAACCGAATTC 733

FIG. 2

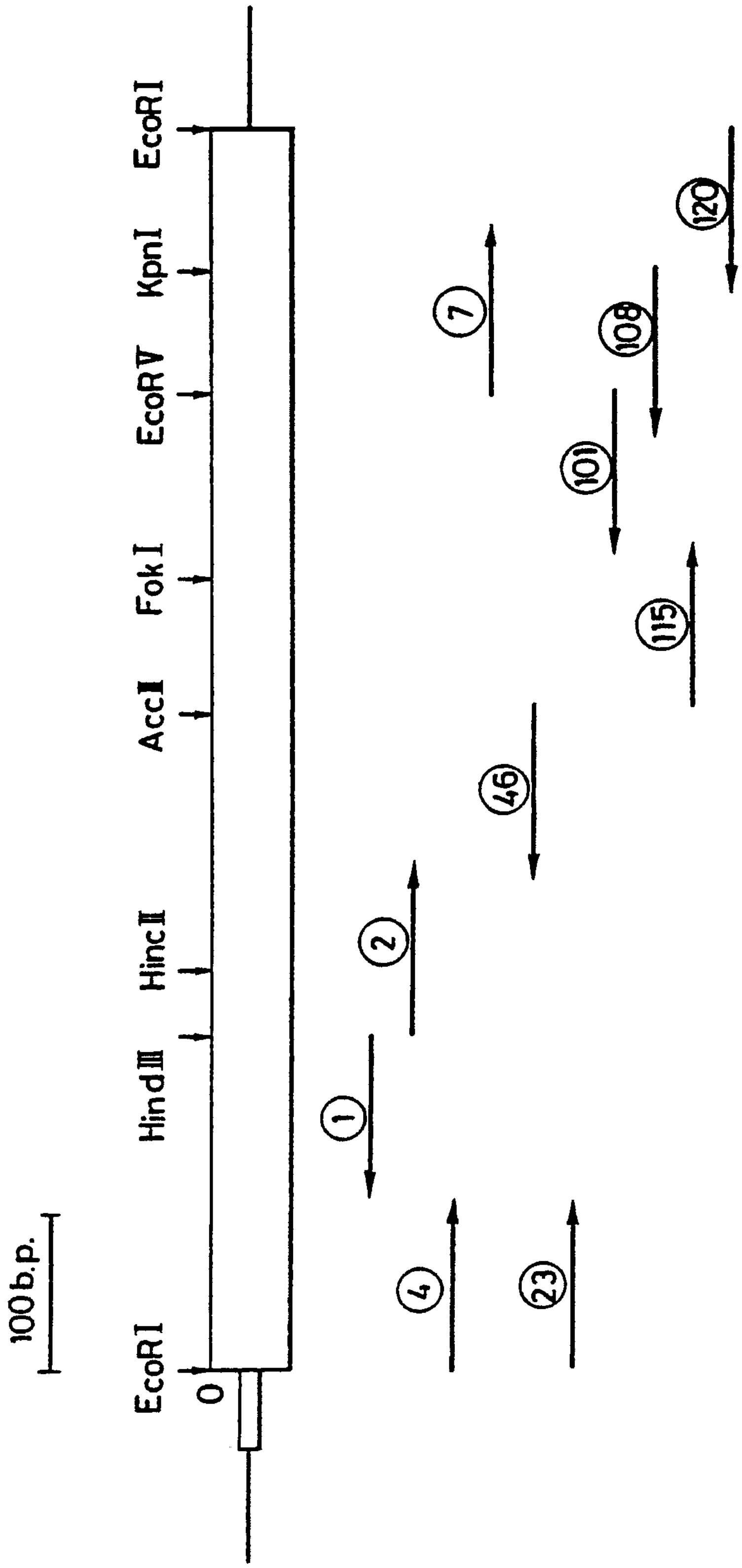
100b.p.
0
EcoRI
HindIII
HincII
AccII
FokI
EcoRV
KpnI
EcoRI
1
2
4
7
23
46
101
108
115
120

European Patent Office

# EUROPEAN SEARCH REPORT

Application Number

EP 90 10 6308

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 301 961 (INSTITUT PASTEUR)<br>* Page 16, example 12; page 17, line 30 - page 19, line 13; claims 1-3,6,13,23-26,29 *<br>--- | 1-7 | C 12 N 15/30<br>A 61 K 39/002 |
| X,O | J. CELL. BIOCHEM. (suppl. 1), no. 10A, 1986, page 145, abstract no. C85; J.L. BURG et al.: "Molecular cloning and analysis of genes encoding Toxoplasma Gondii antigens"<br>* Abstract *<br>--- | 1-7 | |
| P,X | WO-A-8 905 658 (TRANSGENE)<br>* The whole document *<br>--- | 1-7 | |
| P,X | PROC. NATL. ACAD. SCI. USA, vol. 86, October 1989, pages 7537-7541; M.F. CESBRON-DELAUW et al.: "Molecular characterization of a 23-kilodalton major antigen secreted by Toxoplasma gondii"<br>* The whole document *<br>----- | 1-7 | |
| | | | **TECHNICAL FIELDS SEARCHED (Int. Cl.5)** |
| | | | C 12 N<br>A 61 K |

The present search report has been drawn up for all claims

| Place of search | Date of completion of the search | Examiner |
|---|---|---|
| THE HAGUE | 12-07-1990 | SKELLY J.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document